# FASCICULE DE BREVET EUROPEEN

(11) **EP 0 734 319 B1**
(45) Date de publication et mention de la délivrance du brevet: **06.03.2002**
(21) Numéro de dépôt: 95934701.4
(22) Date de dépôt: 13.10.1995
(51) Int. Cl.: B29D 11/02, B29C 45/14, B29C 43/18

(54) **PROCEDE DE REALISATION D'UN IMPLANT INTRAOCULAIRE A OPTIQUE SOUPLE**
VERFAHREN ZUR HERSTELLUNG EINER INTRAOKULARLINSE MIT EINEM FLEXIBLEN OPTISCHEN TEIL
METHOD FOR MAKING AN INTRAOCULAR IMPLANT WITH A SOFT LENS

(30) Priorité: 14.10.1994 FR 9412274; 14.10.1994 FR 9412275
(43) Date de publication de la demande: 02.10.1996
(73) Titulaire: CORNEAL LABORATOIRES, 75012 Paris (FR)
(72) Inventeur: BOS, Gilles, F-74330 La Balme-de-Sillingy (FR); ORTUNO, Angel, F-74330 Choisy (FR); VILLAIN, Franck, F-74000 Annecy (FR)
(74) Mandataire: Dronne, Guy
(86) Numéro de dépôt international: FR9501344
(87) Numéro de publication internationale: WO9611792

(56) Documents cités:
- EP-A- 0 331 457
- FR-A- 1 362 588
- FR-A- 2 510 768
- FR-A- 2 676 357
- FR-A- 2 676 358
- US-A- 4 501 715
- US-A- 5 074 942
- PATENT ABSTRACTS OF JAPAN vol. 6 no. 179 (P-142) [1057] ,14 Septembre 1982 & JP,A,57 094706 (RICOH K.K.) 12 Juin 1982,

## Description

La présente invention a pour objet un procédé pour la fabrication d'implant intraoculaire comportant une optique souple, c'est-à-dire une optique susceptible d'être pliée.

Les implants intraoculaires connaissent un développement important. Ils constituent un système correcteur de la vision humaine qui peut dans un certain nombre de cas se substituer à des lentilles cornéennes ou à des verres correcteurs externes. Un implant intraoculaire se compose essentiellement d'une partie optique de forme générale circulaire ou légèrement ovalisée qui constitue le système optique correcteur proprement dit, et par une partie haptique qui sert à la mise en place, à la fixation et au maintien en position correcte de la partie optique à l'intérieur de l'oeil.

Les implants intraoculaires les plus récents ont une structure monobloc en PMMA, c'est-à-dire que l'optique et la partie haptique sont taillées dans un même bloc de ce matériau. Le plus souvent la partie haptique consiste en deux anses courbes et flexibles qui s'étendent de part et d'autre de la partie optique en étant raccordées à celle-ci en deux points de sa périphérie sensiblement diamétralement opposés.

Pour assurer une correction optique convenable, la partie optique présente un diamètre de l'ordre de 5 à 6 mm. Compte tenu du fait que le matériau utilisé est rigide, du moins dans la partie constituant l'optique, il est nécessaire de pratiquer dans la cornée du patient une incision dont la dimension est au moins égale à 6 mm et en fait supérieure à cette dimension en raison de la présence des parties haptiques.

En outre, il faut souligner que de très nombreux implants de ce type ont été développés, notamment ceux décrits dans les demandes de brevet français Nos. 2 676 358 et 2 676 357, ces implants différant essentiellement par la forme et les caractéristiques dimensionnelles des anses formant la partie haptique, ces caractéristiques étant adaptées notamment au desiderata des praticiens assurant la mise en place de ces implants dans l'oeil des patients. En d'autres termes, la définition en particulier des anses de ces implants, leurs propriétés mécaniques, notamment de flexion, et la stabilité dans le temps de ces caractéristiques mécaniques sont maintenant très bien maîtrisées par les fabriquants d'implants intraoculaires. L'opération d'ablation du cristallin, opération le plus souvent préalable à la mise en place d'un implant, du moins pour un implant en chambre postérieure, nécessitait une incision de la cornée de grande dimension, les implants monoblocs en PMMA étaient très bien adaptés dans la mesure où l'incision pratiquée du fait même de l'opération de la cataracte était de dimension bien suffisante.

L'opération de la cataracte se pratique maintenant le plus souvent selon la technique opératoire dite de phaco-émulsification. Cette technique permet l'ablation du cristallin opaque par l'introduction dans l'oeil d'une sonde à ultrasons disposant d'un système d'irrigation/aspiration. Par l'action combinée des ultrasons et du flux de BSS, on vient retirer le cristallin par émulsification.

Cette technique opératoire présente l'avantage, notamment par rapport aux techniques antérieures, de ne nécessiter que la réalisation d'une incision de dimension réduite dans la cornée grâce à un couteau précalibré à 3,2 mm pour introduire dans l'oeil les instruments nécessaires à cette ablation. On comprend dès lors qu'il est intéressant de disposer d'implants qui puissent être introduits dans l'oeil par l'incision effectuée pour l'opération de phaco-émulsification, c'est-à-dire par une incision dont la longueur est de l'ordre de 3 ou 4 mm.

On comprend également que les implants monoblocs en PMMA étant rigides ne répondent pas à la question posée. C'est pourquoi on a commencé à développer des implants intraoculaires dits souples qui sont réalisés ou du moins dont la partie optique est réalisée à l'aide d'un matériau souple, la partie optique pouvant ainsi être pliée avant son introduction dans l'oeil par l'incision, la partie optique reprenant sa forme initiale après sa mise en place dans l'oeil. Actuellement, deux grands types de produits sont utilisés pour réaliser les optiques souples. Ces produits sont habituellement désignés sous le nom générique, d'une part, d'acryliques souples et, d'autre part, de gel de polysiloxane. Ces produits présentent les propriétés optiques requises pour la réalisation du système optique et par ailleurs sont bio-compatibles.

Lors de la réalisation d'implants intraoculaires à optique souple, l'un des points cruciaux est la réalisation de la partie haptique et, en particulier, la solidarisation de cette partie haptique avec la partie optique.

Parmi les solutions qui ont déjà été utilisées pour résoudre ce problème, on trouve d'une part des solutions dans lesquelles, lors de la réalisation de la partie optique souple, on ménage des orifices dans celle-ci pour venir engager ultérieurement une extrémité de chaque élément de la partie haptique et solidariser cette extrémité avec la partie optique. Une autre solution consiste à réaliser la partie optique par moulage après avoir mis en place dans le moule les éléments de partie haptique de telle façon que les extrémités des parties haptiques constituent l'équivalent d'inserts dans la cavité du moule.

Ces différentes techniques présentent l'inconvénient majeur de ne pas permettre la réalisation de parties haptiques conformes à celles qu'on trouve dans les implants monoblocs en PMMA. En effet, ces parties haptiques sont réalisées à l'aide de fils le plus souvent en polypropylène dont les formes possibles sont limitées. En outre, ces parties haptiques ont des comportements mécaniques qui ont été expérimentés depuis longtemps sans grand succès. En d'autres termes, la longue expérience résultant de la fabrication d'implants monoblocs à partie haptique en PMMA n'est plus utilisable. En outre, dans le premier type de procédé, des opérations supplémentaires sont nécessaires, ce qui accroît bien sûr le coût de la réalisation de l'implant.

Pour résoudre ce problème, on a proposé, dans le EP 331 457, de réaliser l'implant de la manière suivante. On part d'une plaque de PMMA, dans laquelle on réalise des ouvertures dont le diamètre correspond à celui de la partie optique. On remplit les ouvertures avec un monomère MMA qu'on fait polymériser. Enfin, on découpe la plaque de PMMA pour obtenir des ébauches d'implants intraoculaires.

La liaison entre la partie optique et la partie haptique est réalisée par une légère diffusion des matériaux à leur interface.

Un objet de la présente invention est de fournir un procédé de réalisation d'un implant intraoculaire à optique souple qui soit d'une mise en oeuvre simple tout en améliorant la tenue mécanique entre la partie optique et la partie haptique et en permettant d'obtenir une partie haptique en PMMA ou en matériau similaire qui peut prendre toute forme souhaitée, permettant ainsi de bénéficier de l'expérience antérieure des implants intraoculaires monoblocs en PMMA ou matériau similaire.

Pour atteindre ce but, le procédé de réalisation d'un implant intraoculaire comprenant une partie optique définie par une première et une deuxième surface et par une périphérie limitée par des premier et deuxième contours fermés, chacun desdits contours limitant une desdites surfaces, ladite partie optique étant réalisée en un premier matériau souple, et une partie haptique réalisée en un deuxième matériau relativement rigide par rapport audit premier matériau, comprend les étapes suivantes :
on fournit un moule présentant une première et une deuxième partie de moule présentant chacune une face interne définissant une surface limitée par un contour fermé ;
on fournit une plaque du deuxième matériau présentant un évidement central dont la périphérie a des dimensions supérieures ou éventuellement égales à celles de la périphérie de la partie optique envisagée, ladite plaque présentant au moins une portion d'ancrage faisant saillie à l'intérieur dudit évidement ;
on dispose ladite plaque entre lesdites première et deuxième parties de moule de telle manière que les contours fermés desdites parties de moule soient en regard de ladite plaque, lesdites surfaces de parties de moule étant définies de telle manière que le volume limité par lesdites surfaces et ledit évidement de la plaque contienne au moins la forme de la partie optique envisagée ;
on applique avec pression lesdites parties de moule sur ladite plaque, dans cette position lesdites surfaces des parties de moule définissant un volume dont la forme est une enveloppe de la partie optique à réaliser, et on dispose dans le volume défini par au moins ledit évidement et la surface de la partie inférieure du moule un volume dudit premier matériau sous forme liquide ;
on applique audit premier matériau un traitement pour que celui-ci prenne son état solide, par quoi ladite portion d'ancrage constitue un insert à l'intérieur dudit premier matériau ;
on démoule la pièce ainsi obtenue ; et
on usine au moins ladite plaque pour obtenir la forme de partie haptique souhaitée.

Selon un premier mode de mise en oeuvre, dans lequel ledit matériau diminue de volume entre son état liquide et son état solide, on usine ledit premier matériau pour lui donner la forme souhaitée d'optique.

On comprend que, si ce procédé est mis en oeuvre avec un matériau qui peut présenter une réduction de volume significative lors de l'opération de transformation le faisant passer à sa forme finale, la forme définitive de la partie optique devra être obtenue par un usinage ultérieur. En effet, dans le cas d'un matériau présentant un retrait, la cavité du moule définie notamment par les deux parties de moule est telle que, même après retrait, la masse de matériau destinée à constituer la partie optique est une enveloppe de la forme finale de la partie optique.

On comprend également que la plaque du deuxième matériau qui pourra de préférence être du PMMA peut être usinée par les techniques utilisées antérieurement pour la réalisation des implants intraoculaires monoblocs réalisés en un matériau du type PMMA. En d'autres termes, le fabricant pourra bénéficier de la longue expérience de la définition des formes et dimensions des parties haptiques, notamment des anses haptiques réalisées antérieurement.

Dans une variante de réalisation, on peut, de plus, utiliser deux matériaux qui sont choisis pour réaliser une interpénétration de réseaux entre ces deux matériaux lors de la transformation du premier matériau dans le moule, cette interpénétration ne devant pas modifier la rigidité du premier matériau.

La réalisation par moulage de la partie optique peut se faire de deux manières différentes. Dans un premier cas, les deux parties du moule sont appliquées avec pression sur la plaque et le matériau, sous forme liquide, est injecté dans la cavité du moule. Dans un deuxième cas, avant la mise en place de la partie supérieure du moule, on dépose en excès dans l'évidement formé par la partie inférieure du moule et l'orifice de la plaque le matériau destiné à former la partie optique, puis on met en place la partie supérieure du moule et on applique avec pression les deux parties du moule sur la plaque.

Le choix du procédé se fait en fonction de l'état initial liquide ou relativement visqueux du matériau.

On comprend également que ce premier mode de mise en oeuvre du procédé permet, dans le cas où le premier matériau présente un faible retrait et est injecté, de limiter les opérations d'usinage pour obtenir la forme d'implant finale à l'usinage de la plaque du deuxième matériau qui est de préférence du PMMA, cette technique étant parfaitement maîtrisée par les fabricants d'implants intraoculaires et à la suppression dans la partie optique des traces d'injection.

Un deuxième mode de mise en oeuvre du procédé est défini dans la revendication 16.

On comprend que ce deuxième mode de mise en oeuvre du procédé est mis en oeuvre avec un matériau ne présentant pas une réduction de volume significative lors de l'opération de transformation pour passer de sa forme de transformation à sa forme finale.

On comprend également que la forme finale de la partie optique est directement définie par la forme des deux parties du moule. On comprend également que la plaque du deuxième matériau qui pourra de préférence être du PMMA peut être usinée par les techniques utilisées antérieurement pour la réalisation des implants intraoculaires monoblocs réalisés en un matériau du type PMMA. En d'autres termes, le fabricant pourra bénéficier de la longue expérience de la définition des formes et dimensions des parties haptiques, notamment des anses haptiques réalisées antérieurement.

Dans une première variante de réalisation, on peut, de plus, utiliser deux matériaux qui sont choisis pour réaliser une interpénétration de réseaux entre ces deux matériaux lors de la transformation du premier matériau dans le moule, cette interpénétration ne devant pas modifier la rigidité du premier matériau.

Selon une deuxième variante de mise en oeuvre, on peut, de plus, apprêter la périphérie de l'évidement central de la plaque pour réaliser l'adhérence des premier et deuxième matériaux lors de l'opération de moulage. Ces deux variantes de réalisation peuvent être utilisées ensemble.

On comprend également que ce deuxième mode de mise en oeuvre du procédé permet de limiter les opérations d'usinage pour obtenir la forme d'implant finale à l'usinage de la plaque du deuxième matériau qui est de préférence du PMMA, cette technique étant parfaitement maîtrisée par les fabricants d'implants intraoculaires.

D'autres caractéristiques et avantages de la présente invention apparaîtront mieux à la lecture de la description qui suit de plusieurs modes de réalisation de l'invention donnés à titre d'exemples non limitatifs. La description se réfère aux figures annexées sur lesquelles :
- la figure 1 est une vue en coupe verticale d'un moule dans sa première position lors de l'élaboration de l'implant selon un premier mode de mise en oeuvre ;
- la figure 2 est une vue similaire montrant la mise en place de la deuxième partie du moule ;
- la figure 3 montre le moule en position fermée ;
- la figure 4 illustre le produit obtenu à la fin de l'étape de moulage et montre en partie l'opération d'usinage de la partie haptique ;
- les figures 5 et 6 montrent en coupe verticale une variante de réalisation du moule pour la mise en oeuvre du procédé selon une variante du premier mode de mise en oeuvre et selon un deuxième mode de mise en oeuvre ; et
- la figure 7 est une vue analogue à celle de la figure 4 pour le deuxième mode de mise en oeuvre de l'invention.

Avant de décrire en détails des modes préférés de mise en oeuvre du procédé de fabrication de l'implant intraoculaire selon l'invention, on va en exposer le principe.

Le principe du procédé, selon son premier mode de mise en oeuvre de l'invention, consiste à fournir une plaque d'un deuxième matériau destiné à constituer la partie haptique qui est par exemple du PMMA. Cette plaque comporte un évidement central dont la périphérie a au moins les dimensions de la périphérie de la partie optique. En d'autres termes, la périphérie de la partie optique à réaliser doit être contenue dans l'évidement central de la plaque pour des raisons qui seront explicitées ultérieurement. Cette plaque a des dimensions suffisantes pour permettre l'usinage de l'ensemble de la partie haptique selon les techniques en elles-mêmes connues. Cette plaque est disposée entre les deux parties d'un moule qui définissent en particulier des surfaces enveloppes des deux surfaces limitant la partie optique. On introduit dans la cavité constituée par l'évidement de la plaque et les deux parties du moule un premier matériau sous forme liquide qui présente les propriétés optiques requises et qui peut être transformé pour atteindre un état final solide. Ce matériau peut présenter un phénomène de retrait lors de cette transformation. Après avoir réalisé la transformation du matériau et avoir démoulé le produit ainsi obtenu, il suffit d'usiner la plaque pour ne laisser subsister de celle-ci que la partie haptique désirée et le matériau destiné à constituer la partie optique pour lui donner la forme voulue. Typiquement, dans le cas plus particulièrement considéré, il s'agit de deux anses identiques, ces anses pouvant être usinées exactement comme dans le cas de la réalisation d'implants intraoculaires monoblocs en PMMA.

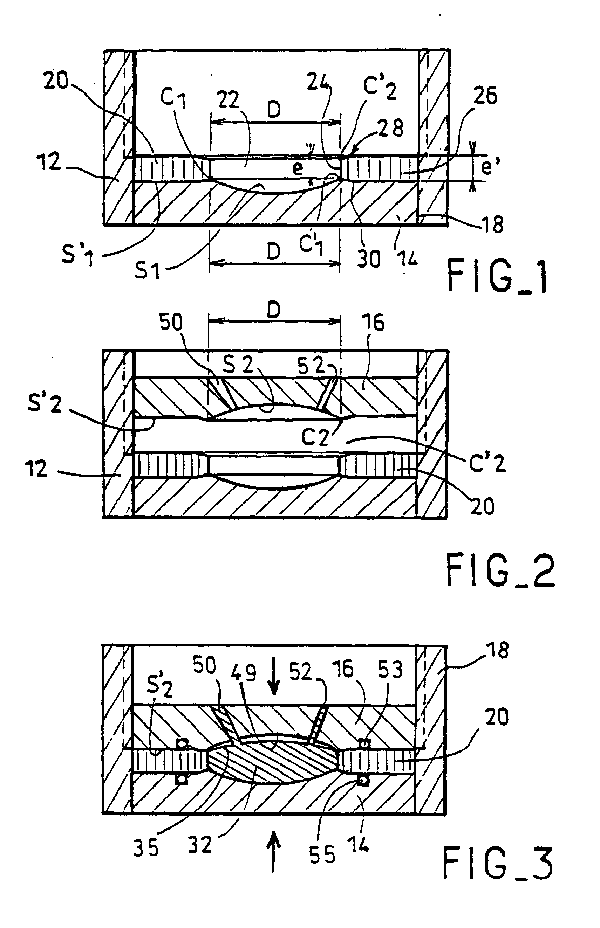

En se référant maintenant plus particulièrement aux figures 1 à 4, on va décrire en détails un premier mode de mise en oeuvre du procédé de fabrication de l'implant intraoculaire.

Comme on l'a déjà indiqué, l'implant intraoculaire est essentiellement réalisé par une opération de moulage. Le moule est essentiellement constitué par une bague annulaire cylindrique 12, par une partie inférieure 14 et par une partie supérieure 16. La partie inférieure 14 présente une portion centrale dont la face supérieure est limitée par une portion de surface S1 qui est une enveloppe d'une des faces de l'optique de l'implant à réaliser. La portion de surface Si est limitée par un contour fermé C1. Au-delà du contour fermé C1, la partie inférieure 14 du moule comporte une portion périphérique dont la face supérieure est, par exemple, définie par une portion de cône ou une surface tronconique S'1 qui peut être en retrait par rapport au contour C1 ou être plane. Comme le montre la figure, la périphérie externe 18 de la partie de moule 14 a le méme diamètre que la face interne de la bague cylindrique 12.

Selon l'invention, pour réaliser l'implant, on vient placer à l'intérieur de la bague 12 et entre la partie inférieure 14 et la partie supérieure 16 une plaque 20 qui est réalisée dans un matériau apte à former la partie haptique de l'implant. Typiquement, pour les raisons déjà indiquées précédemment, ce matériau est de préférence du PMMA. Cependant on pourrait utiliser d'autres types de matériaux. Le contour externe de la plaque 20 a le même diamètre que le diamètre interne de la bague 12. La plaque 20 comporte un orifice central 22 dont le diamètre D est au moins égal au diamètre de la partie optique à réaliser. L'orifice 22 est donc entouré par une périphérie 24, cette périphérie étant limitée elle-même par un premier contour fermé inférieur C'1 et par un deuxième contour fermé supérieur C'2. Dans le cas particulier de la figure, ces contours sont circulaires, de diamètre au moins égal à D et ils sont disposés dans des plans parallèles. Pour réaliser d'autres types d'optiques, on pourrait avoir des dispositions différentes. En outre, il faut remarquer que le contour inférieur C'1 de la plaque 20, de préférence, coïncide exactement avec le contour fermé C1 de la partie inférieure du moule 14. La périphérie 24 de l'orifice 22 présente une épaisseur e qui est au moins égale à l'épaisseur du contour de la partie optique à réaliser. La partie courante de la plaque 20, qui porte la référence 26, présente une épaisseur e' sensiblement supérieure e de la paroi 24. Cette épaisseur e' est suffisante pour permettre le taillage ultérieur des parties haptiques. On sait en effet que le plus souvent les parties haptiques ou anses haptiques présentent une certaine angulation par rapport au plan de l'optique de l'implant. De préférence également, la périphérie 24 de l'orifice 22 est raccordée à la partie courante 26 de la plaque par deux surfaces 28 et 30 qui peuvent être planes.

Dans l'étape suivante représentée par la figure 2, on vient disposer à l'intérieur de la bague cylindrique 12 la deuxième partie 16 du moule. Cette partie 16 comporte une face inférieure présentant une partie centrale qui définit une surface S2 limitée par un contour fermé C2. La surface S2 est une enveloppe de la deuxième face de la partie optique à réaliser et le contour C2, de préférence, coïncide avec le deuxième contour supérieur C'2 de la plaque. La partie 16 du moule comporte également une zone périphérique qui est de préférence limitée par une surface conique ou tronconique S'2. En outre, la partie supérieure 16 comporte au moins un orifice d'injection 50 et au moins un évent 52. Le ou les orifices d'injection ainsi que le ou les évents pouraient également être disposés dans la partie inférieure du moule ou dans la plaque.

La partie supérieure du moule 16 est alors abaissée de telle manière que son contour fermé C2 vienne en regard du contour C'2 de la plaque et on applique une pression sur les parties 14 et 16 du moule de telle façon que la cavité interne 49 limitée par les surfaces Si et S2 et par la périphérie 24 de l'orifice 22 de la plaque 20 soit étanche. Pour améliorer encore l'étanchéité, il est possible d'interposer deux joints annulaires d'étanchéité entre la plaque 20 et les parties 14 et 16 du moule. C'est ce qu'on a représenté en 53 et 55 sur la figure 3.

On injecte alors par l'orifice 50 dans la cavité 49 le matériau 32 destiné à constituer la partie optique. Ce matériau est sous forme liquide. Dans un exemple préféré, ce matériau est un hydrogel qui, dans cette phase, est sous la forme liquide d'une solution de monomères. On sait que ce matériau présente un retrait non négligeable lors de sa transformation ou "prise" pour passer de l'état liquide à l'état solide. Le matériau est soumis au traitement convenable pour provoquer sa solidification. On comprend que la bague 12 sert seulement au guidage et au positionnement relatif des deux parties du moule de telle façon que les contours fermés C1, C2, C'1 et C'2 soient effectivement en regard. La bague n'a aucune fonction d'étanchéité. L'étanchéité de l'empreinte du moule est réalisée par le contact avec pression des contours fermés. Comme on l'a déjà indiqué, l'étanchéité peut être améliorée par des joints. Selon la nature du matériau utilisé, le traitement peut consister en un traitement thermique ou en un bombardement par des photons ou par des particules telles que des électrons. Dans le cas du matériau considéré dans cet exemple, celui-ci présente un retrait. C'est ce qu'on a représenté en 35 sur la figure 3.

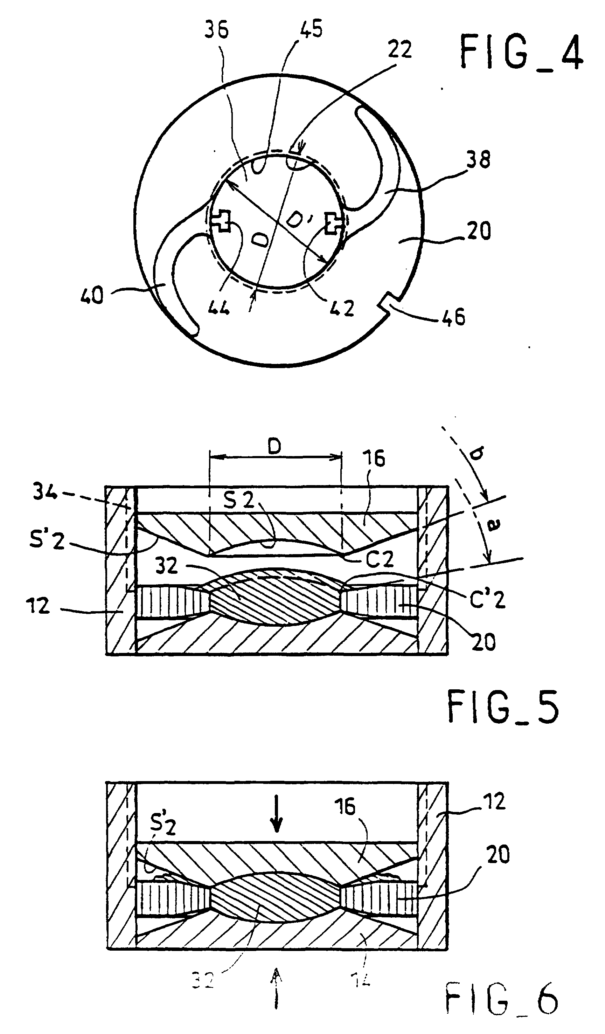

Lorsque ce traitement est terminé, il suffit de démouler la pièce ainsi obtenue. C'est ce qui est représenté sur la figure 4. La pièce obtenue est donc constituée par la plaque 22 et par la zone optique 36 constituée par le matériau 32.

Pour assurer la liaison mécanique entre la périphérie de l'optique 36 et la périphérie de l'orifice 22 de la plaque 20, c'est-à-dire entre la partie haptique et la partie optique, différentes techniques sont envisageables selon la nature des matériaux utilisés. Il est possible de choisir des matériaux qui, lors de la transformation du premier matériau destiné à constituer l'optique, réalisent entre ces deux matériaux un phénomène d'interpénétration de réseaux. Si la plaque 20 est en PMMA, on peut utiliser comme premier matériau du PHEMA. Cette interpénétration s'accompagne le plus souvent d'une rigidification de la périphérie de la partie optique 36.

Selon l'invention, une autre solution ou une solution complémentaire consiste à prévoir à l'intérieur de l'orifice 22 de la plaque 20 deux extensions de cette plaque telles que 42 et 44 qui joueront le rôle d'insert lors de la réalisation de la partie optique par moulage. Pour terminer la pièce, il faut procéder à l'usinage du bloc de matériau destiné à constituer la partie optique et de la plaque 20. Cet usinage consiste d'abord à donner à la partie optique 36 la forme requise en usinant ses deux surfaces S1 et S2. Il consiste également à usiner la plaque 20 afin de définir les anses 38 et 40 dans l'exemple particulier décrit. Cet usinage comporte de plus, dans le cas où il y a interpénétration de réseau entre les deux matériaux, l'étape de découper la périphérie de la partie optique 36 selon la ligne 45 pour éliminer la zone d'interpénétration qui peut être rigide. Cette découpe présente le diamètre D' qui est celui de la partie optique à réaliser. Cest la raison pour laquelle le diamètre D de l'évidement central 22 de la plaque est supérieur à celui de la partie optique.

Une solution complémentaire consiste à apprêter la surface de l'orifice 22 ou des extensions 42 et 44 de façon à obtenir une adhésion entre les deux matériaux.

Il faut ajouter que même si le premier matériau présente un retrait négligeable et qu'il n'y a pas de réaction entre les deux matériaux, il peut être intéressant de taillier le matériau constituant l'optique, par exemple pour supprimer les traces d'injection.

Dans tous les cas, on obtient un implant intraoculaire comportant :
- d'une part, des anses taillées dans la partie 20, typiquement dans du PMMA,
- d'autre part, une optique usinée dans le matériau 32 injecté à l'intérieur du moule.

L'optique sera souple, soit parce que le matériau choisi est hydrophile et, dans ce cas, c'est son hydratation qui aura un rôle plastifiant ; soit parce que le matériau choisi a une température de transition vitreuse inférieure à la température ambiante et, dans ce cas, il aura été usiné en dessous de cette température.

Les figures 5 et 6 illustrent une variante de moule pour la réalisation d'un implant intraoculaire, cette variante concernant la façon dont le matériau est disposé à l'intérieur de la cavité du moule, cette introduction n'étant plus réalisée par injection.

Dans cette variante du premier mode de mise en oeuvre, la partie inférieure 14 du moule et la bague annulaire 12 sont identiques à celles qui sont représentées sur la figure 1. Il est donc inutile de les décrire à nouveau.

Dans l'étape suivante représentée par la figure 5, on vient déposer dans la cavité constituée par la surface Si de la partie inférieure 14 du moule et par l'orifice 22 de la plaque 20 un volume 32 du matériau destiné à constituer l'optique. Dans un exemple préféré, typiquement ce matériau peut être un acrylique qui, dans cette phase, est sous la forme liquide d'une solution de monomères. Cette solution aura pu être prépolymérisée pour obtenir un sirop ayant une viscosité compatible avec la dépose d'une goutte 32 de ce produit. On sait que ce matériau présente un retrait non négligeable lors de sa transformation ou "prise" pour passer de l'état liquide à l'état solide. Le volume 32 déposé est sensiblement supérieur au volume de l'optique à réaliser. On vient alors disposer à l'intérieur de la bague cylindrique 12 la deuxième partie 16 du moule. Cette partie 16 comporte une face inférieure présentant une partie centrale qui définit une surface S2 limitée par un contour fermé C2. La surface S2 est une enveloppe de la deuxième face de la partie optique à réaliser et le contour C2, de préférence, coïncide avec le deuxième contour supérieur C2 de la plaque. La partie 16 du moule comporte également une zone périphérique qui est de préférence limitée par une surface conique ou tronconique S'2.

La partie supérieure du moule 16 est alors abaissée de telle manière que son contour fermé C2 vienne en regard du contour C'2 de la plaque Durant cette opération, l'excès de matériau 32 est chassé dans le volume résiduel annulaire s'étendant entre la plaque et la surface conique S'2 de la partie supérieure 16 du moule. L'abaissement est réalisé de telle manière qu'aucune bulle d'air ne reste dans le matériau. Pour faciliter la sortie de l'excès du matériau 32, l'angle au sommet a de la portion tronconique 28 de la plaque est supérieur à l'angle au sommet b de la surface tronconique S'2 de la partie supérieure 16 du moule. Eventuellement, on peut prévoir dans la bague cylindrique 12 et plus précisément dans sa face interne des canaux verticaux tels que 34 d'évacuation de l'air. Dans le moule on maintient sous pression l'empilage constitué par la partie inférieure 14, la plaque 20 et la partie supérieure 16. On comprend que la bague 12 sert seulement au guidage et au positionnement relatif des deux parties du moule de telle façon que les contours fermés C1, C2, C'1 et C'2 soient effectivement en regard. La bague n'a aucune fonction d'étanchéité. L'étanchéité de l'empreinte du moule est réalisée par le contact avec pression des contours fermés. En outre, la surface de contact étant très réduite, on évite la formation, dans la pièce moulée, de parties parasites dans les deux plans de joint. On soumet alors l'ensemble du moule à un traitement pour provoquer la transformation du matériau 32 depuis son état liquide initial jusqu'à son état final stable à la température ambiante. Selon la nature du matériau utilisé, le traitement peut consister en un traitement thermique ou en un bombardement par des photons ou par des particules telles que des électrons. Dans le cas du matériau considéré dans cet exemple, celui-ci présente un retrait. Cest ce qu'on a représenté en 35 sur la figure 6.

Lorsque ce traitement est terminé, il suffit de démouler la pièce ainsi obtenue. C'est ce qui est représenté sur la figure 4. La pièce obtenue est donc constituée par la plaque 22 et par la zone optique 36 constituée par le matériau 32. On procède alors à l'usinage de la partie optique et de la partie haptique comme indiqué précédemment. Cette solution n'est intéressante que lorsque le matériau servant à réaliser la partie optique présente dans son état initial une certaine viscosité. Cette condition est remplie dans le cas des gels de polysiloxanes. Dans le cas des acryliques, on peut réaliser un début de polymérisation du produit pour l'amener dans l'état visqueux requis.

Dans le cas où le matériau injecté présente un retrait important, par exemple dans le cas d'un acrylique, l'usinage qui devra être réalisé ultérieurement est identique à celui qui a été décrit en liaison avec la figure 4.

Dans le cas d'un matériau présentant un retrait négligeable, il est possible, en donnant aux parties 14 et 16 du moule la forme exacte de l'optique à réaliser et en limitant les plans de joint du moule à des contacts de surface limitée correspondant aux contours fermés C1, C2, C'1, C'2, d'obtenir directement la partie optique par moulage par injection. L'usinage de la partie optique se limitera à la suppression des traces, sur la surface de la pièce, du ou des points d'injection et du ou des évents. Il faut bien sûr que ce matériau, en outre, soit usinable à la température ambiante ou à une température proche de la température ambiante, ce qui n'est pas le cas des gels de silicone.

Dans l'exemple plus particulièrement décrit, l'optique à réaliser est limitée par les surfaces S1 et S2 qui sont des portions de calotte sphérique afin de définir la lentille. En conséquence, les contours fermés C1, C2, C'1, C'2 sont circulaires. Il va de soi que les surfaces S1 et S2 peuvent être quelconques, notamment concaves ou convexes, et présenter le rayon de courbure souhaité. Il va également de soi que l'une des surfaces S1 pourrait en fait être plane. Il va encore de soi que, dans le cas où l'on veut réaliser un implant, par exemple du type multifocal, les surfaces S1 et S2 pourraient être plus complexes. On comprend que la forme des surfaces S1 et S2 ne modifie en rien les différentes étapes du procédé.

Dans le cas où le matériau utilisé présente un retrait non négligeable, il est nécessaire de procéder à un usinage de la partie optique. La forme de l'empreinte dans le moule, et l'importance des surfaces de contact entre les deux parties du moule et la plaque ne sont donc pas critiques.

Lorsqu'on accepte un retrait significatif du matériau et qu'on recherche un effet d'interpénétration de réseaux entre les matériaux constituant la partie optique et la partie haptique, on peut utiliser avantageusement les matériaux désignés communément sous les termes de pHEMA, d'hydrogel ou de tout autre copolymère d'acrylique souple, la plaque 20 étant elle réalisée en PMMA.

Les documents EP-A-0 485 197 et EP-A-0 514 096 décrivent des acryliques souples utilisables pour la mise en oeuvre de l'invention. Les documents US-A-4 997 442, US-A-5 133 745, WO-A-90/09768 et EP-A-0 492 126 décrivent des hydrogels également utilisables pour la mise en oeuvre de l'invention.

Le principe du procédé, selon l'invention, consiste à fournir une plaque d'un deuxième matériau destiné à constituer la partie haptique qui est par exemple du PMMA. Cette plaque comporte un évidement central dont la périphérie a exactement la forme de la périphérie de la partie optique. Cette plaque a des dimensions suffisantes pour permettre l'usinage de l'ensemble de la partie haptique selon les techniques en elles-mêmes connues. Cette plaque est disposée entre les deux parties d'un moule qui définissent en particulier les deux surfaces limitant la partie optique. On introduit dans la cavité constituée par l'évidement de la plaque et les deux parties du moule un premier matériau sous forme liquide qui présente les propriétés optiques requises et qui peut être transformé pour atteindre un état final solide. Ce matériau doit présenter un phénomène de retrait lors de cette transformation sensiblement négligeable. Après avoir réalisé la transformation du matériau et avoir démoulé le produit ainsi obtenu, il suffit d'usiner la plaque pour ne laisser subsister de celle-ci que la partie haptique désirée.

Ce deuxième mode de mise en oeuvre du procédé utilise un moule identique à celui qui a été décrit en liaison avec les figures 1, 4 et 5 et on ne le redécrira donc pas en détails.

Selon le deuxième mode de mise en oeuvre du procédé, pour réaliser l'implant, on vient placer à l'intérieur de la bague 12 et au-dessus de la partie inférieure 14 une plaque 20 qui est réalisée dans un matériau apte à former la partie haptique de l'implant. Typiquement, pour les raisons déjà indiquées précédemment, ce matériau est de préférence du PMMA. Cependant on pourrait utiliser d'autres types de matériaux. Le contour externe de la plaque 20 a le même diamètre que le diamètre interne de la bague 12. La plaque 20 comporte un orifice central 22 dont le diamètre D est égal au diamètre de la partie optique à réaliser. L'orifice 22 est donc entouré par une périphérie 24, cette périphérie étant limitée elle-même par un premier contour fermé inférieur C'1 et par un deuxième contour fermé supérieur C'2. Dans le cas particulier de la figure, ces contours sont circulaires, de diamètre D et ils sont disposés dans des plans parallèles. Pour réaliser d'autres types d'optiques, on pourrait avoir des dispositions différentes. En outre, il faut remarquer que le contour inférieur C'1 de la plaque 20 coïncide exactement avec le contour fermé C1 de la partie inférieure du moule 14. La périphérie 24 de l'orifice 22 présente une épaisseur e qui est strictement égale à l'épaisseur du contour de la partie optique à réaliser. La partie courante de la plaque 20, qui porte la référence 26, présente une épaisseur e' sensiblement supérieure'à l'épaisseur e de la paroi 24. Cette épaisseur e' est suffisante pour permettre le taillage ultérieur des parties haptiques. On sait en effet que le plus souvent les parties haptiques ou anses haptiques présentent une certaine angulation par rapport au plan de l'optique de l'implant. De préférence également, la périphérie 24 de l'orifice 22 est raccordée à la partie courante 26 de la plaque par deux surfaces 28 et 30 qui, dans le cas de la figure, sont des troncs de cône mais qui, plus généralement selon les formes à réaliser, pourraient être des portions de surface conique.

Dans l'étape suivante représentée par la figure 5, on vient déposer dans la cavité constituée par la surface S1 de la partie inférieure 14 du moule et par l'orifice 22 de la plaque 20 un volume 32 du matériau destiné à constituer l'optique. Typiquement ce matériau peut être un gel de silicone qui, dans cette phase, est sous une forme liquide. Le volume 32 déposé est supérieur au volume de l'optique à réaliser. On vient alors disposer à l'intérieur de la bague cylindrique 12 la deuxième partie 16 du moule. Cette partie 16 comporte une face inférieure présentant une partie centrale qui définit une surface S2 limitée par un contour fermé C2. La surface S2 est identique à la deuxième face de la partie optique à réaliser et le contour C2 coïncide avec le deuxième contour supérieur C'2 de la plaque. La partie 16 du moule comporte également une zone périphérique qui est de préférence limitée par une surface conique ou tronconique S'2.

La partie supérieure du moule 16 est alors abaissée de telle manière que son contour fermé C2 vienne exactement en regard du contour C'2 de la plaque. Durant cette opération, l'excès de matériau 32 est chassé dans le volume résiduel annulaire s'étendant entre la plaque et la surface conique S'2 de la partie supérieure 16 du moule. L'abaissement est réalisé de telle manière qu'aucune bulle d'air ne reste dans le matériau. Pour faciliter la sortie de l'excès du matériau 32, l'angle au sommet a de la portion tronconique 28 de la plaque est supérieur à l'angle au sommet b de la surface tronconique S'2 de la partie supérieure 16 du moule. Eventuellement, on peut prévoir dans la bague cylindrique 12 et plus précisément dans sa face interne des canaux verticaux tels que 34 d'évacuation de l'air. Dans le moule on maintient sous pression l'empilage constitué par la partie inférieure 14, la plaque 20 et la partie supérieure 16. On comprend que la bague 12 sert seulement au guidage et au positionnement relatif des deux parties du moule de telle façon que les contours fermés C1, C2, C'1 et C'2 soient effectivement en regard. La bague n'a aucune fonction d'étanchéité. L'étanchéité de l'empreinte du moule est réalisée par le contact avec pression des contours fermés. En outre, la surface de contact étant très réduite, on évite la formation, dans la pièce moulée, de parties parasites dans les deux plans de joint. On soumet alors l'ensemble du moule à un traitement pour provoquer la transformation du matériau 32 depuis son état liquide initial jusqu'à son état final stable à la température ambiante. Il peut être assimilable à un gel et donc être souple parce que sa température de transition vitreuse est plus basse que la température ambiante ou bien il peut être hydrophile et devenir souple après absorption d'eau qui joue le rôle de plastifiant. Selon la nature du matériau utilisé, le traitement peut consister en un traitement thermique ou en un bombardement par des photons ou par des particules telles que des électrons.

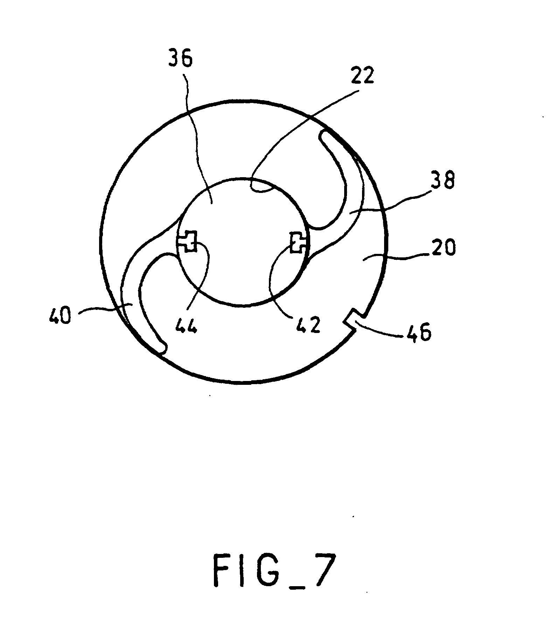

Lorsque ce traitement est terminé, il suffit de démouler la pièce ainsi obtenue. C'est ce qui est représenté sur la figure 7. La pièce obtenue est donc constituée par la plaque 22 et par la zone optique 36 constituée par le matériau 32. Pour terminer la fabrication de l'implant intraoculaire, il suffit de découper dans la plaque 20 par des techniques d'usinage classiques la partie haptique souhaitée. Dans le cas de la figure 7, cette partie haptique consiste en deux anses identiques diamétralement opposés référencés 38 et 40.

Pour assurer la liaison mécanique entre la périphérie de l'optique 36 et la périphérie de l'orifice 22 de la plaque 20, c'est-à-dire entre la partie haptique et la partie optique, différentes techniques sont envisageables selon la nature des matériaux utilisés. Il est possible de choisir des matériaux qui, lors de la transformation du premier matériau destiné à constituer l'optique, réalisent entre ces deux matériaux un phénomène d'interpénétration de réseaux. Il faut cependant que cette interpénétration ne s'accompagne pas d'une rigidification de la périphérie de la partie optique 36.

Selon l'invention on prévoit à l'intérieur de l'orifice 22 de la plaque 20 deux extensions de cette plaque telles que 42 et 44 qui joueront le rôle d'insert lors de la réalisation de la partie optique par moulage. Par ailleurs, il n'y aucune liaison mécanique entre les deux matériaux, c'est-à-dire entre la plaque et la partie optique 36. Lors du découpage des anses 38 et 40, il est bien sûr nécessaire que la partie de ces anses au contact de la périphérie de la partie optique comporte les extensions 42 et 44. Pour réaliser cette condition, il suffit de prévoir sur la périphérie de la plaque 20 un élément d'indexage 46 permettant de positionner angulairement les outils d'usinage des anses 38 et 40.

Une solution complémentaire consiste à apprêter au préalable la périphérie 24 de l'orifice 22 de la plaque de façon à réaliser une adhérence entre le matériau constituant la partie haptique et le matériau constituant la partie optique 36. Dans tous les cas, après découpage et usinage de la plaque 20 pour réaliser les anses, on obtient un implant intraoculaire qui comporte une partie optique 36 réalisée en un matériau souple et une partie haptique constituée par exemple les anses 38 et 40 qui présentent exactement les mêmes propriétés mécaniques et exactement les mêmes formes que les anses des implants intraoculaires antérieurs monoblocs réalisés par exemple en PMMA. L'apprêtage peut être effectué sur les extensions 38 et 40 destinées à servir d'inserts pour compléter la liaison entre les deux matériaux.

Dans l'exemple plus particulièrement décrit, l'optique à réaliser est limitée par les surfaces S1 et S2 qui sont des portions de calotte sphérique afin de définir la lentille. En conséquence, les contours fermés C1, C2, C'1, C'2 sont circulaires. Il va de soi que les surfaces Si et S2 peuvent être quelconques, notamment concaves ou convexes, et présenter le rayon de courbure souhaité. Il va également de soi que l'une des surfaces Si pourrait en fait être plane. Il va encore de soi que, dans le cas où l'on veut réaliser un implant, par exemple du type multifocal, les surfaces S1 et S2 pourraient être plus complexes. On comprend que la forme des surfaces S1 et S2 ne modifie en rien les différentes étapes du procédé.

De préférence, le matériau utilisé pour l'anneau 20 est le PMMA.

De préférence, le matériau utilisé pour réaliser la partie optique, dans ce deuxième mode de mise en oeuvre du procédé, est un gel de silicone qui présente toutes les propriétés requises et notamment un très faible retrait lors de sa transformation. Les gels de silicone se polymérisent avec une variation de volume négligeable. Les gels de silicone provenant de la réticulation de polydimethyl-siloxanes, polymethylphenylsiloxanes, polydiphenylsiloxanes et leurs copolymères conviennent (la présence de fonctions phényles permettant d'augmenter l'indice de réfraction du matériau). Les copolymères de silicone avec par exemple les acryliques peuvent convenir.

Des polysiloxanes utilisables pour la mise en oeuvre de l'invention sont notamment décrits dans les documents suivants : EP-A-0 226 400, EP-A-0 556 040, WO-A-93/16660 et FR-A-2 587 896.

Le document EP-A-0 335 312 décrit la formulation comprenant un polysiloxane avec terminaisons vinyles et des groupements phényles pour augmenter l'indice de réfraction et un polysiloxane ayant des fonctions hydrures ainsi qu'un absorbeur UV qui n'est que dispersé dans la silicone. Mis à part le filtre UV dispersé, c'est le même type de formulation qui est utilisé dans la fabrication de toutes les implants intraoculaires en silicone. On préférera un filtre UV lié de manière covalente à la silicone.

On peut également utiliser des matériaux thermoplastiques à très faible retrait et présentant les propriétés optiques et de biocompatiblité requises. Ces matériaux présentent l'avantage de pouvoir être injectés dans l'empreinte du moule.

On peut enfin utiliser des résines acryliques prépolymérisées pour obtenir un sirop de viscosité suffisante et de retrait faible pendant la polymérisation finale. Les polymères acryliques concernés soit appartiennent à la famille des hydrogels, soit ont une température de transition vitreuse (Tv) inférieure à la température ambiante. Dans tous les cas, on ajoutera un filtre UV dans le polymère avant réticulation. On préfèrera un filtre UV lié par covalence à ce polymère.

Il faut souligner que, dans le cas où le matériau constituant l'optique est un gel de silicone, le procédé selon le deuxième mode de mise en oeuvre est particulièrement intéressant. En effet, ce matériau ne peut être usiné à la température ambiante ou même à une température proche de la température ambiante.

En outre, pour ce qui est de la liaison entre la partie optique et la partie haptique, on prévoit les extensions 40 et 42 de la plaque 20, éventuellement combinées aux autres modes de liaison. En effet, dans le produit final, ces extensions assurent un ancrage mécanique très fiable des extrémités des anses haptiques dans la partie optique.

## Revendications

1. Procédé de réalisation d'un implant intraoculaire comprenant une partie optique définie par une première et une deuxième surface et par une périphérie limitée par des premier et deuxième contours fermés, chacun desdits contours limitant une desdites surfaces, ladite partie optique étant réalisée en un premier matériau souple, et une partie haptique réalisée en un deuxième matériau relativement rigide par rapport audit premier matériau, ledit procédé comprenant les étapes suivantes :
on fournit un moule présentant une première (14) et une deuxième (16) partie de moule présentant chacune une face interne (S1, S2) définissant une surface limitée par un contour fermé (C1, C2) ;
on fournit une plaque (20) du deuxième matériau présentant un évidement central (22) dont la périphérie a des dimensions supérieures ou éventuellement égales à celles de la périphérie de la partie optique envisagée, ladite plaque présentant au moins une portion d'ancrage (42, 44) faisant saillie à l'intérieur dudit évidement ;
on dispose ladite plaque (20) entre lesdites première et deuxième parties (14, 16) de moule de telle manière (20) que les contours fermés desdites parties de moule soient en regard de ladite plaque, lesdites surfaces de parties de moule étant définies de telle manière que le volume limité par lesdites surfaces et ledit évidement de la plaque contienne au moins la forme de la partie optique envisagée ;
on applique avec pression lesdites parties de moule sur ladite plaque, dans cette position lesdites surfaces des parties de moule définissant un volume dont la forme est une enveloppe de la partie optique à réaliser, et on dispose dans le volume défini par au moins ledit évidement et la surface de la partie inférieure du moule un volume dudit premier matériau sous forme liquide ;
on applique audit premier matériau un traitement pour que celui-ci prenne son état solide, par quoi ladite portion d'ancrage constitue un insert à l'intérieur dudit premier matériau ;
on démoule la pièce ainsi obtenue ; et
on usine au moins ladite plaque pour obtenir la forme de partie haptique souhaitée.

2. Procédé selon la revendication 1, dans lequel ledit premier matériau diminue de volume lors de son passage de l'état liquide à l'état solide **caractérisé en ce que** :
on usine en outre ledit premier matériau pour lui donner la forme souhaitée d'optique.

3. Procédé selon la revendication 2, **caractérisé en ce qu'**au moins une desdites parties du moule et/ou ladite plaque comporte au moins un orifice d'injection, **en ce qu'**on applique avec pression lesdites parties du moule sur ladite plaque de telle manière que l'évidement de ladite plaque et les surfaces des parties du moule forment une cavité étanche, et **en ce qu'**on injecte ledit premier matériau sous forme liquide dans ladite cavité à travers ledit orifice d'injection.

4. Procédé selon la revendication 3, **caractérisé en ce qu'**au moins une desdites parties du moule et/ou ladite plaque comprend au moins un évent.

5. Procédé selon la revendication 2, **caractérisé en ce que** ledit premier matériau présente une diminution de volume lors de sa transformation de l'état liquide à l'état solide et **en ce que** les surfaces des parties de moule sont des enveloppes des surfaces définissant la partie optique.

6. Procédé selon l'une quelconque des revendications 2 à 5, **caractérisé en ce que** l'usinage dudit premier matériau comprend l'usinage desdites première et deuxième surfaces définissant la partie optique.

7. Procédé selon l'une quelconque des revendications 2 à 6, **caractérisé en ce que** ledit premier matériau présente avec le deuxième matériau un phénomène d'interpénétration de réseau à leur interface et **en ce que** les dimensions de la périphérie de l'évidement de ladite plaque sont au moins égales à celles de la périphérie de la partie optique.

8. Procédé selon la revendication 7, **caractérisé en ce que** l'usinage dudit premier matériau comprend la suppression, à la périphérie dudit premier matériau, de la zone annulaire ayant été le siège de phénomènes d'interpénétration de réseau entre les deux matériaux à l'exception des zones de raccordement de la partie haptique sur la périphérie de la partie optique.

9. Procédé selon l'une quelconque des revendications 2 à 4, **caractérisé en ce qu'**on utilise un premier matériau présentant une diminution de volume négligeable lors de sa transformation de l'état liquide à l'état solide, **en ce que** lesdites surfaces des parties de moule sont identiques aux première et deuxième surfaces définissant la partie optique et la périphérie de l'évidement de ladite plaque est identique à la périphérie de la partie optique.

10. Procédé selon la revendication 9, **caractérisé en ce que** l'usinage dudit premier matériau consiste au moins dans l'enlèvement du ou des points d'injection dudit matériau dans ladite cavité et du ou des évents.

11. Procédé selon l'une quelconque des revendications 2 à 5, **caractérisé en ce que** ledit deuxième matériau est du PMMA et le premier matériau est un composé acrylique hydrophile.

12. Procédé selon l'une quelconque des revendications 2 à 5, **caractérisé en ce que** ledit deuxième matériau est du PMMA et le premier matériau est un composé acrylique dont la température de transition vitreuse est suffisamment inférieure à la température ambiante.

13. Procédé selon la revendication 2, **caractérisé en ce que** ledit premier matériau est disposé dans ladite cavité avant l'application desdites parties de moule sur ladite plaque, le volume dudit matériau étant supérieur à celui de la cavité résultant de l'application des parties de moule sur ladite plaque.

14. Procédé selon la revendication 13, **caractérisé en ce que** lesdits premier et deuxième matériaux sont choisis pour réaliser une interpénétration de réseau entre lesdits matériaux lors de la transformation dudit premier matériau, par quoi on obtient une solidarisation de la périphérie de l'évidement de ladite plaque avec la périphérie de ladite partie optique et desdites portions d'ancrage dans ledit premier matériau.

15. Procédé selon l'une quelconque des revendications 1 à 14, **caractérisé en ce qu'**on apprête lesdites portions d'ancrage pour réaliser l'adhérence des premier et second matériaux,

16. Procédé selon la revendication 1 pour la réalisation d'un implant intraoculaire comprenant une partie optique (36) définie par deux portions de surfaces (S1, S2) et par une périphérie limitée par deux contours fermés (C'1, C'2), chaque contour fermé limitant une desdites surfaces, **caractérisé en ce qu'**il comprend les étapes suivantes:
on fournit une partie inférieure de moule (14) présentant une surface supérieure dont la partie centrale (S1) correspondant à ladite première portion de surface limitée par ledit premier contour fermé (C1) et dont la partie périphérique (S1') est en retrait par rapport audit premier contour ;
on fournit une plaque (20) réalisée avec ledit deuxième matériau présentant un évidement central (22) dont la périphérie (24) est limitée par lesdits premier (C'1) et deuxième (C'2) contours fermés, ladite plaque présentant au moins une portion d'ancrage (42, 44) faisant saillie à l'intérieur dudit évidement ;
on dispose ladite plaque en regard de ladite partie inférieure du moule de telle manière que ledit contour fermé de ladite première partie soit en regard du premier contour fermé de ladite plaque ;
on dispose au-dessus de ladite plaque une deuxième partie de moule (16) présentant une surface inférieure dont la partie centrale correspond à ladite deuxième portion de surface limitée par ledit deuxième contour fermé (C2) et dont la partie périphérique (S'2) est en retrait par rapport audit deuxième contour fermé, de telle manière que les deuxièmes contours fermés de ladite plaque et de la surface inférieure de la deuxième partie de moule soient en regard ;
on introduit dans l'espace défini par au moins une desdites surfaces et par ledit évidement un volume (32) du premier matériau sous forme liquide au moins égal au volume de ladite partie optique ;
on applique avec pression lesdites parties de moule de part et d'autre de ladite plaque de telle manière que respectivement les premiers contours fermés et les deuxièmes contours fermés entrent en contact mutuel par quoi ledit premier matériau remplit la totalité du volume limité par ledit évidement et lesdites portions de surface, l'excès éventuel dudit premier matériau étant chassé hors dudit volume;
on soumet ledit premier matériau à un traitement pour assurer sa prise et l'amener dans son état final stable, par quoi ladite portion d'ancrage forme un insert à l'intérieur dudit premier matériau ;
on démoule la pièce ainsi obtenue ; et
on usine ladite plaque pour réaliser la forme souhaitée de la partie haptique (38, 40).

17. Procédé selon la revendication 16, **caractérisé en ce que** lesdits premier et deuxième matériaux sont choisis pour réaliser une interpénétration de réseau entre lesdits matériaux lors de la transformation dudit premier matériau, par quoi on obtient une solidarisation de la périphérie de l'évidement de ladite plaque avec la périphérie de ladite partie optique et desdites portions d'ancrage dans ledit premier matériau.

18. Procédé selon la revendication 16, **caractérisé en ce qu'**on apprête lesdites portions d'ancrage pour réaliser l'adhérence des premier et second matériaux.

19. Procédé selon l'une quelconque des revendications 16 à 18, **caractérisé en ce que** ledit deuxième matériau est du PMMA.

20. Procédé selon l'une quelconque des revendications 16 à 19, **caractérisé en ce que** ledit premier matériau est un polysiloxane.

21. Procédé selon l'une quelconque des revendications 16 à 19, **caractérisé en ce que** ledit premier matériau est un matériau thermoplastique.

22. Procédé selon l'une quelconque des revendications 16 à 19, **caractérisé en ce que** ledit premier matériau est un polymère acrylique de la famille des hydrogels.

23. Procédé selon l'une quelconque des revendications 16 à 19, **caractérisé en ce que** ledit premier matériau est un polymère acrylique ayant une température de transition vitreuse inférieure à la température ambiante.

24. Procédé selon l'une quelconque des revendications 11, 12 et 20 à 23, **caractérisé en ce que** ledit deuxième matériau comprend un agent de filtrage anti-UV lié par covalence.

25. Procédé selon l'une quelconque des revendications 16 à 24, **caractérisé en ce que** lesdites première et deuxième portions de surface sont des calottes sphériques et **en ce que** lesdits premier et deuxième contours fermés sont deux cercles égaux et parallèles entre eux.

26. Procédé selon la revendication 25, **caractérisé en ce que** ladite plaque présente une partie courante d'épaisseur e', **en ce que** la périphérie dudit évidement présente une épaisseur e < e', la partie courante étant raccordée à la périphérie dudit évidement par au moins une portion de surface conique (28, 30) de raccordement d'angle au sommet a.

27. Procédé selon la revendication 26, **caractérisé en ce que** la partie périphérique de la partie de moule en regard de la face de la plaque présentant la surface conique de raccordement présente, au-delà dudit contour fermé circulaire une portion de surface conique présentant un angle au sommet b inférieur à l'angle au sommet a.

## Claims

1. A method of making an intraocular implant comprising a lens portion defined by first and second surfaces and by a periphery bounded by first and second closed contours, each of said contours bounding one of said surfaces, said lens portion being made of a first flexible material, and a haptic portion being made of a second material that is rigid relative to the first material, said method comprising the following steps:
a mould is provided having first and second mould parts (14, 16) each having an inside face (S1, S2) defining a surface that is bounded by a closed contour (C1, C2);
a plate (20) made of a second material is provided that has a central recess (22) whose periphery is of dimensions greater than or optionally equal to those of the periphery of the intended lens portion, said plate having at least one anchoring portion (42, 44) projecting inside said recess;
said plate (20) is disposed between said first and second mould parts (14, 16) in such a manner (20) that the closed contours of said mould parts face said plate, said surfaces of the mould parts being defined in such a manner that the volume bounded by said surfaces and
said recess of the plate contains at least the shape of the intended lens portion;
said mould parts are applied with pressure against said plate, with said mould parts defining in said position a volume whose shape is an envelope of the lens portion to be made, and a volume of said first material in liquid form is disposed in the volume defined at least by said recess and the surface of the bottom part of the mould;
treatment is applied to said first material so that it takes on its solid state, whereby said anchoring portion forms an insert inside said first material;
the piece obtained in this way is unmoulded; and
at least said plate is machined so as to obtain the desired shape of haptic portion.

2. A method according to claim 1, in which said first material decreases in volume on passing from the liquid state to the solid state, and **characterised in that**:
said first material is also machined so as to give the desired lens shape thereto.

3. A method according to claim 2, **characterised in that** at least one of said mould parts and/or said plate includes at least one injection orifice, **in that** said mould parts are applied with pressure against said plate in such a manner that the recess in said plate and the surfaces of the mould parts form a sealed cavity, and **in that** said first material is injected in liquid form into said cavity through said injection orifice.

4. A method according to claim 3, **characterised in that** at least one of said mould parts and/or said plate includes at least one vent.

5. A method according to claim 2, **characterised in that** said first material decreases in volume on transforming from the liquid state to the solid state, and **in that** the surfaces of the mould part are envelopes of the surfaces that define the lens portion.

6. A method according to any one of claims 2 to 5, **characterised in that** the machine of said first material includes machining said first and second surfaces that define the lens portion.

7. A method according to any one of claims 2 to 6, **characterised in that** said first material co-operates with said second material to present a phenomenon of network interpenetration at their interface, and **in that** the dimensions of the periphery of the recess in said plate are not less than those of the lens portion.

8. A method according to claim 7, **characterised in that** the machining of said first material includes eliminating from the periphery of said first material the annular zone in which network interpenetration phenomena have taken place between the two materials, with the exception of the zones where the haptic portions are coupled to the periphery of the lens portion.

9. A method according to any one of claims 2 to 4, **characterised in that** a first material is used that presents negligible decrease in volume on being transformed from the liquid state to the solid state, **in that** said surfaces of the mould parts are identical to the first and second surfaces defining the lens portion, and the periphery of the recess in said plate is identical to the periphery of the lens portion.

10. A method according to claim 9, **characterised in that** the machining of said first material consists at least in removing the injection point(s) of said material into said cavity, and the vent(s).

11. A method according to any one of claims 2 to 5, **characterised in that** said second material is PMMA and the first material is a hydrophilic acrylic compound.

12. A method according to any one of claims 2 to 5, **characterised in that** the second material is PMMA and the first material is an acrylic compound whose glass-transition temperature is far enough below ambient temperature.

13. A method according to claim 2, **characterised in that** said first material is disposed in said cavity prior to said mould parts being applied against said plate, the volume of said material being greater than that of the cavity that results from applying the mould parts against said plate.

14. A method according to claim 13, **characterised in that** said first and second materials are selected to enable network interpenetration to take place between said materials during transformation of said first material, whereby the periphery of the recess in said plate is secured to the periphery of said lens portion, and to the periphery of said anchor portions in said first material.

15. A method according to any one of claims 1 or 14, **characterised in that** said anchor portions are prepared to provide adhesion between the first and second materials.

16. A method according to claim 1, for making an intraocular implant comprising a lens portion (36) defined by two surface portions (S1, S2) and by a periphery bounded by two closed contours (C'1, C'2), each closed contour itself bounding one of said surfaces, the method being **characterised in that** it comprises the following steps:
a bottom mould part (14) is provided having a top surface with a central portion (S1) corresponding to said first portion of surface bounded by said first closed contour (C1) and whose peripheral portion (S1') is set back relative to said first contour;
a plate (20) is provided that is made of said second material and that has a central recess (22) whose periphery (24) is bounded by said first and second closed contours (C'1, C'2), said plate having at least one anchoring portion (42, 44) projecting inside said recess;
said plate is disposed facing said bottom part of the mould in such a manner that said closed contour of said first portion faces the first closed contour of said plate;
a second mould part (16) is disposed above said plate, said second mould portion having a bottom surface whose central portion corresponds to said second surface portion bounded by said second closed contour (C2) and whose peripheral portion (S'2) is set back from said second closed contour, in such a manner that the second closed contours of said plate and of the bottom surface of said second mould part face each other;
a volume (32) of the first material in liquid form is inserted into the space defined by at least one of said surfaces and by said recess, said volume being not less than the volume of said lens portion;
said mould parts are applied with pressure on either side of said plate in such a manner that said first and second closed contours come respectively into mutual contact whereby said first material fills all of the volume bounded by said recess and said surface portions, any excess of said first material being expelled from said volume;
said first material is subjected to treatment to cause it to set and to bring it to its stable final state, whereby said anchoring portion forms an insert inside said first material;
the piece obtained in this way is unmoulded; and
said plate is machined to achieve the desired shape for the haptic portion (38, 40).

17. A method according to claim 16, **characterised in that** said first and second materials are selected so as to achieve network interpenetration between said materials during transformation of said first material, thereby securing the periphery of the recess of said plate to the periphery of said lens portion, and said anchor portions in said first material.

18. A method according to claim 16, **characterised in that** said anchor portions are prepared to provide adhesion between said first and second materials.

19. A method according to any one of claims 16 to 18, **characterised in that** said second material is PMMA.

20. A method according to any one of claims 16 to 19, **characterised in that** said first material is a polysiloxane.

21. A method according to any one of claims 16 to 19, **characterised in that** said first material is a thermoplastic material.

22. A method according to any one of claims 16 to 19, **characterised in that** said first material is an acrylic polymer from the hydrogel family.

23. A method according to any one of claims 16 to 19, **characterised in that** said first material is an acrylic polymer having a glass-transition temperature lower than ambient temperature.

24. A method according to any one of claims 11, 12, and 20 to 23, **characterised in that** said second material comprises an anti-UV filtering agent that is covalently bonded.

25. A method according to any one of claims 16 to 24, **characterised in that** said first and second surface portions are spherical caps, and **in that** said first and second closed contours are two equal and mutually parallel circles.

26. A method according to claim 25, **characterised in that** said plate has a main portion of thickness e', **in that** the periphery of said recess has a thickness e < e', the main portion being connected to the periphery of said recess via at least one coupling conical surface portion (28, 30) having an angle at the apex of a.

27. A method according to claim 26, **characterised in that** the peripheral portion of the mould part facing the face of the plate which has the coupling conical surface portion presents, beyond said circular closed contour, a conical surface portion having an angle at the apex b that is smaller than the angle at the apex a.

## Patentansprüche

1. Verfahren zur Herstellung einer Intraokularlinse mit einem optischen Teil, welches durch eine erste und eine zweite Oberfläche sowie durch eine mittels erster und zweiter geschlossener Konturen begrenzte Peripherie definiert wird, wobei jede der Konturen eine der Oberflächen begrenzt, das optische Teil aus einem ersten, flexiblen Material hergestellt ist und mit einem haptischen Teil, das aus einem im Verhältnis zu dem ersten Material relativ unelastischen Material hergestellt ist, wobei das Verfahren die folgenden Schritte aufweist:
- dass eine Form vorgesehen wird, die ein erstes (14) und zweites (16) Formteil mit jeweils einer inneren Fläche (S1, S2) aufweist, die eine durch eine geschlossene Kontur begrenzte Oberfläche (C1, C2) definieren;
- dass eine aus dem zweiten Material bestehende Platte (20) vorgesehen wird, die eine zentrale Ausnehmung (22) aufweist, deren Peripherie größere oder unter Umständen gleiche Abmessungen wie die Peripherie des betrachteten optischen Teils hat, wobei die Platte wenigstens einen Verankerungsabschnitt (42, 44) aufweist, der in das Innere der Ausnehmung ragt;
- dass die Platte (20) zwischen dem ersten und zweiten Formteil (14, 16) derart angeordnet wird, dass die geschlossenen Konturen der Formteile der Platte gegenüberliegen und die Oberflächen der Formteile derart definiert sind, dass das durch die Oberflächen und die Vertiefung der Platte begrenzte Volumen wenigsten die Form des beabsichtigten optischen Teils umfasst;
- dass die Formteile unter Druck auf die Platte aufgebracht werden, wobei in dieser Stellung die Oberflächen der Formteile ein Volumen bestimmen, dessen Form eine Hüllkurve des herzustellenden optischen Teils ist und wenigstens in das durch die Ausnehmung und die Oberfläche des unteren Teils definierte Volumen ein Volumen des ersten Materials in flüssiger Form gefüllt wird;
- dass auf das erste Material eine Behandlung angewandt wird, aufgrund derer dieses seinen festen Zustand annimmt, wodurch der Verankerungsabschnitt einen Einsatz im Inneren des ersten Materials bildet;
- dass das so erhaltene Stück aus der Form genommen und zumindest die Platte bearbeitet, um die gewünschte Form des haptischen Teils zu erhalten.

2. Verfahren nach Anspruch 1, bei dem das erste Material infolge des Übergangs vom flüssigen in den festen Zustand sein Volumen verringert, **dadurch gekennzeichnet, dass** überdies das erste Material bearbeitet wird, um es in die für die Optik gewünschte Form zu bringen.

3. Verfahren nach Anspruch 2, **dadurch gekennzeichnet, dass** wenigstens eines der Formteile und/oder die Platte wenigstens eine Einspritzöffnung aufweisen, die Formteile mit Druck auf die Platte derart aufgebracht werden, dass die Ausnehmung der Platte und die Oberflächen der Formteile einen dichten Hohlraum bilden und das erste Material in flüssiger Form durch die Einspritzöffnung in den Hohlraum eingespritzt wird.

4. Verfahren nach Anspruch 3, **dadurch gekennzeichnet, dass** zumindest eines der Formteile und/oder die Platte wenigstens eine Lüftungsöffnung aufweisen.

5. Verfahren nach Anspruch 2, **dadurch gekennzeichnet, dass** das erste Material bei seiner Umbildung vom flüssigen in den festen Zustand eine Volumenverringerung mit sich bringt und die Oberflächen der Formteile Hüllkurven der das optische Teil definierenden Oberflächen sind.

6. Verfahren nach einem der Ansprüche 2 bis 5, **dadurch gekennzeichnet, dass** die Bearbeitung des ersten Materials eine Bearbeitung der ersten und zweiten das optische Teil definierenden Oberflächen beinhaltet.

7. Verfahren nach einem der Ansprüche 2 bis 6, **dadurch gekennzeichnet, dass** das erste Material mit dem zweiten Material an deren Schnittstelle ein Phänomen gegenseitiger Netzdurchdringung aufweisen und die Abmessungen der Peripherie der Ausnehmung der Platte wenigstens gleich groß im Verhältnis zu denen der Peripherie des optischen Teils sind.

8. Verfahren nach Anspruch 7, **dadurch gekennzeichnet, dass** die Behandlung des ersten Materials einen Abtrag der ringförmigen Zone, die der Ort des Phänomens gegenseitiger Netzdurchdringung war, an der Peripherie des ersten Materials mit Ausnahme der Verbindungszonen zwischen dem haptischen und dem optischen Teil umfasst.

9. Verfahren nach einem der Ansprüche 2 bis 4, **dadurch gekennzeichnet, dass** ein erstes Material mit unwesentlicher Volumenverringerung während seiner Umwandlung vom festen in den flüssigen Zustand verwendet wird, die Oberflächen der Formteile mit den ersten und zweiten das optische Teil definierenden Oberflächen identisch ist und die Peripherie der Ausnehmung der Platte mit der Peripherie des optischen Teils identisch ist.

10. Verfahren nach Anspruch 9, **dadurch gekennzeichnet, dass** die Behandlung des ersten Materials wenigstens in der Beseitigung des oder aus dem Material bestehenden Angusspunkte in dem Hohlraum und der oder den Lüftungsöffnungen besteht.

11. Verfahren nach einem der Ansprüche 2 bis 5, **dadurch gekennzeichnet, dass** das zweite Material aus Polymethylmethacrylat (PMMA) besteht und das erste Material eine hydrophile Acrylverbindung ist.

12. Verfahren nach einem der Ansprüche 2 bis 5, **dadurch gekennzeichnet, dass** das zweite Material aus PMMA besteht und das erste Material eine Acrylverbindung ist, deren Glasübergangstemperatur hinreichend geringer als Raumtemperatur ist.

13. Verfahren nach Anspruch 2, **dadurch gekennzeichnet, dass** das erste Material vor der Anbringung der Formteile auf der Platte in dem Hohlraum angeordnet wird, wobei das Volumen des Materials aufgrund der Anbringung der Formteile auf der Platte größer als das des Hohlraums ist.

14. Verfahren nach Anspruch 13, **dadurch gekennzeichnet, dass** die ersten und zweiten Materialien zur Erreichung gegenseitiger Netzdurchdringung zwischen den Materialien während der Umwandlung des ersten Materials ausgewählt sind, wodurch ein Zusammenschluss der Peripherie der Ausnehmung der Platte mit der Peripherie des optischen Teils und den Verankerungsabschnitten in dem ersten Material erhalten wird.

15. Verfahren nach einem der Ansprüche 1 bis 14, **dadurch gekennzeichnet, dass** die Verankerungsabschnitte zur Herstellung der Haftung der ersten und zweiten Materialien ausgerüstet sind.

16. Verfahren nach Anspruch 1 zur Herstellung einer Intraokularlinse mit einem optischen Teil (36), das durch zwei Oberflächenabschnitte (S1, S2) und durch eine mittels zweier geschlossener Konturen (C'1, C'2) begrenzte Peripherie definiert wird, wobei jede geschlossene Kontur eine Oberfläche begrenzt, **dadurch gekennzeichnet, dass** das Verfahren die folgenden Schritte aufweist:
- dass ein Formunterteil vorgesehen wird, das eine obere Oberfläche aufweist, deren mittlerer Teil dem ersten durch die erste geschlossene Kontur begrenzten Oberflächenabschnitt entspricht und deren peripherer Teil (S1') im Verhältnis zu der ersten Kontur zurückgesetzt ist;
- dass eine aus dem zweiten Material hergestellte Platte (20) vorgesehen wird, die eine mittlere Ausnehmung (22) aufweist, deren Peripherie durch die ersten (C'1) und zweiten (C'2) geschlossenen Konturen begrenzt wird und die Platte wenigstens einen Verankerungsabschnitt (42, 44) aufweist, der in das Innere der Ausnehmung ragt;
- dass die Platte dem Formunterteil gegenüberstehend derart angeordnet wird, dass die geschlossene Kontur des ersten Teils der ersten geschlossenen Kontur der Platte gegenübersteht;
- dass oberhalb der Platte ein zweites Formteil (16) angeordnet wird, welches eine untere Oberfläche aufweist, deren mittleres Teil der zweiten durch die zweite geschlossene Kontur (C2) begrenzten Oberfläche entspricht und deren peripheres Teil (S'2) bezüglich der zweiten geschlossenen Kontur zurückgesetzt ist, derart, dass die zweiten geschlossenen Konturen der Platte und der unteren Oberfläche des zweiten Formteils sich gegenüberstehen;
- dass in-den wenigstens durch eine der Oberflächen und die Ausnehmung definierten Raum (32) ein Volumen des ersten Materials in flüssiger Form eingeführt wird, welches wenigstens dem Volumen des optischen Teils entspricht;
- dass die Formteile unter Druck zu beiden Seiten der Platte derart aufgebracht werden, dass die ersten und zweiten geschlossenen Konturen in gegenseitigen Kontakt treten, wodurch das erste Material das durch die Ausnehmung und die Oberflächenabschnitte begrenzte Volumen vollständig ausfüllt und ein etwaiger Überschuss des ersten Materials aus dem Volumen herausgetrieben wird;
- dass das erste Material einer Behandlung unterzogen wird um dessen Abbinden und die Beförderung in den stabilen Endzustand sicherzustellen, wodurch der Verankerungsabschnitt einen Einsatz im Inneren des ersten Materials bildet;
- dass das so erhaltene Stück aus der Form genommen wird; und
- dass die Platte zum Erhalt der gewünschten Form des haptischen Teils bearbeitet wird.

17. Verfahren nach Anspruch 16, **dadurch gekennzeichnet, dass** die ersten und zweiten Materialien zur Erreichung gegenseitiger Netzdurchdringung zwischen den Materialien während der Umwandlung des ersten Materials ausgewählt sind, wodurch ein Zusammenschluss der Peripherie der Ausnehmung der Platte mit der Peripherie des optischen Teils und den Verankerungsabschnitten in dem ersten Material erhalten wird.

18. Verfahren nach Anspruch 16, **dadurch gekennzeichnet, dass** die Verankerungsabschnitte zur Herstellung der Haftung der ersten und zweiten Materialien vorbereitet sind.

19. Verfahren nach einem der Ansprüche 16 bis 18, **dadurch gekennzeichnet, dass** das zweite Material aus PMMA besteht.

20. Verfahren nach einem der Ansprüche 16 bis 19, **dadurch gekennzeichnet, dass** das erste Material ein Polysiloxan ist.

21. Verfahren nach einem der Ansprüche 16 bis 19, **dadurch gekennzeichnet, dass** das erste Material ein thermoplastisches Material ist.

22. Verfahren nach einem der Ansprüche 16 bis 19, **dadurch gekennzeichnet, dass** das erste Material ein Acrylpolymer aus der Familie der Hydrogele ist.

23. Verfahren nach einem der Ansprüche 16 bis 19, **dadurch gekennzeichnet, dass** das erste Material ein Acrylpolymer mit einer unter Raumtemperatur liegenden Glasübergangstemperatur ist.

24. Verfahren nach einem der Ansprüche 11, 12 und 20 bis 23, **dadurch gekennzeichnet, dass** das zweite Material einen kovalent gebundenen Anti-UV-Filterwirkstoff aufweist.

25. Verfahren nach einem der Ansprüche 16 bis 24, d**adurch gekennzeichnet**, dass die ersten und zweiten Oberflächenabschnitte sphärische Kalotten sind und dass die ersten und zweiten geschlossenen Konturen zwei gleiche und zueinander parallele Kreise sind.

26. Verfahren nach Anspruch 25, **dadurch gekennzeichnet, dass** die Platte ein schwimmendes Teil der Dicke e' aufweist, die Peripherie der Ausnehmung eine Dicke e < e' aufweist und das schwimmende Teil mit der Peripherie der Ausnehmung durch wenigstens einen konischen Oberflächenverbindungsabschnitt (28, 30) mit einem maximalen Winkel a verbunden ist.

27. Verfahren nach Anspruch 26, **dadurch gekennzeichnet, dass** das der den konischen Oberflächenverbindungsabschnitt darstellenden Fläche der Platte gegenüberstehende periphere Teil des Formteils über die geschlossene kreisförmige Kontur hinaus einen konischen 0berflächenabschnitt hat, der einen maximalen Winkel b aufweist, der kleiner als der maximale Winkel a ist.
